# EUROPEAN PATENT APPLICATION

(11) **EP 4 302 864 A1**
(43) Date of publication of application: **10.01.2024**
(21) Application number: 22926069.0
(22) Date of filing: 30.11.2022
(51) Int. Cl.: B01D 63/06, B01D 71/02, C07C 29/152, C07C 31/04, B01D 53/22

(54) **REACTOR MODULE, LIQUID FUEL SYNTHESIS METHOD, SEPARATION MEMBRANE MODULE, AND SEPARATION METHOD**

(30) Priority: 08.02.2022 JP 2022017956; 25.08.2022 JP 2022134439
(71) Applicant: NGK Insulators, Ltd., Nagoya-shi, Aichi 467-8530 (JP)
(72) Inventor: NAKAGAWA, Kosuke, Nagoya-shi, Aichi 467-8530 (JP); IIDA, Kazuki, Nagoya-shi, Aichi 467-8530 (JP); KAN, Hirofumi, Nagoya-shi, Aichi 467-8530 (JP); TORII, Atsushi, Nagoya-shi, Aichi 467-8530 (JP); SHIOMI, Makoto, Nagoya-shi, Aichi 467-8530 (JP); SHIBAGAKI, Yukinari, Nagoya-shi, Aichi 467-8530 (JP); SHIMIZU, Katsuya, Nagoya-shi, Aichi 467-8530 (JP)
(74) Representative: Müller-Boré & Partner Patentanwälte PartG mbB
(86) International application number: PCT/JP2022/044149
(87) International publication number: WO 2023/153053

(57) **Abstract**

A reactor (1) includes a second flow path (12) on a permeation side of a separation membrane (30). The second flow path (12) includes an inflow port (d1) open to a first space (P1) between a first seal portion (4) and a flow rate adjustment unit (6), and an outflow port (d2) open to a second space (P2) between a second seal portion (5) and a flow rate adjustment unit (6). A housing (3) includes a sweep gas supply port (3a) for supplying a sweep gas to the first space (P1) and a sweep gas exhaust port (3b) for discharging the sweep gas from the second space (P2). In a side view of the reactor (1), a direction in which the sweep gas flows from the first space (P1) to the second space (P2) via the flow rate adjustment unit (6) is the same as a direction in which the sweep gas flows through the second flow path (12).

## Description

### TECHNICAL FIELD

The present invention relates to a reactor module, a liquid fuel synthesis method, a separation membrane module, and a separation method.

### BACKGROUND ART

In recent years, reactors have been developed that can improve, in a conversion reaction of a raw material gas containing hydrogen and carbon oxide to a liquid fuel such as methanol and ethanol (specifically, a fuel that is in a liquid state at normal temperature and pressure), conversion efficiency by separating water vapor that is generated together with the liquid fuel.

For example, Patent Literature 1 discloses a tubular reactor provided with a separation membrane permeable to water vapor, which is one of the products of the conversion reaction, a non-permeation side flow path through which a raw material gas flows, and a permeation side flow path through which a sweep gas flows.

### CITATION LIST

### Patent Literature

Patent Literature 1: JP 2018-8940A

### SUMMARY

### TECHNICAL PROBLEM

According to the reactor disclosed in Patent Literature 1, since reaction heat generated due to the conversion reaction can be removed by causing the sweep gas to flow through the permeation side flow path, the conversion efficiency can be further improved.

However, in the reactor disclosed in Patent Literature 1, since the sweep gas flows through only the permeation side flow path in the reactor, there is a limitation in removing the reaction heat.

Also, in a separation membrane module provided with a separation filter, in some cases, in order to control the separation membrane permeable to a desired component contained in a mixed fluid to an appropriate temperature, the separation membrane is to be cooled or heated by causing the sweep gas to flow through the permeation side flow path.

An object of the present invention is to provide a reactor module, a liquid fuel synthesis method, a separation membrane module, and a separation method, capable of efficiently controlling a temperature.

### SOLUTION TO PROBLEM

A reactor module according to the present invention includes a monolith-type reactor extending in a longitudinal direction, a housing configured to house the reactor, an annular first seal portion configured to seal a space between the housing and a first end portion of the reactor, and an annular second seal portion configured to seal a space between the housing and a second end portion of the reactor, and an air-permeable annular flow rate adjustment unit disposed between the first seal portion and the second seal portion in the longitudinal direction. The reactor includes a separation membrane permeable to a product of a conversion reaction of a raw material gas containing hydrogen and carbon oxide to a liquid fuel, a first flow path on a non-permeation side of the separation membrane, and a second flow path on a permeation side of the separation membrane. The second flow path includes an inflow port open to a first space between the first seal portion and the flow rate adjustment unit, and an outflow port open to a second space between the second seal portion and the flow rate adjustment unit. The housing includes a supply port for supplying a sweep gas to the first space, and an exhaust port for discharging the sweep gas from the second space. In a side view of the reactor, a direction in which the sweep gas flows from the first space to the second space via the flow rate adjustment unit is the same as a direction in which the sweep gas flows through the second flow path.

### ADVANTAGEOUS EFFECTS

According to the present invention, it is possible to provide a reactor module, a liquid fuel synthesis method, a separation membrane module, and a separation method, capable of efficiently controlling a temperature.

### BRIEF DESCRIPTION OF DRAWINGS

FIG. 1 is a perspective view of a reactor 1 according to an embodiment.
FIG. 2 is a cross-sectional view taken along A-A in FIG. 1.
FIG. 3 is a cross-sectional view taken along B-B in FIG. 1.
FIG. 4 is a cross-sectional view taken along C-C in FIG. 2.
FIG. 5 is a transparent side view of a reactor module according to the embodiment.
FIG. 6 is a cross-sectional view of the reactor module according to the embodiment.
FIG. 7 is a cross-sectional view of the reactor module according to the embodiment.
FIG. 8 is a cross-sectional view of a flow rate adjustment unit according to Modification 3.

### DESCRIPTION OF EMBODIMENTS

Embodiments of the present invention will be described with reference to the drawings. However, the drawings are schematic, and ratio or the like of dimensions may differ from an actual one.

### Reactor 1

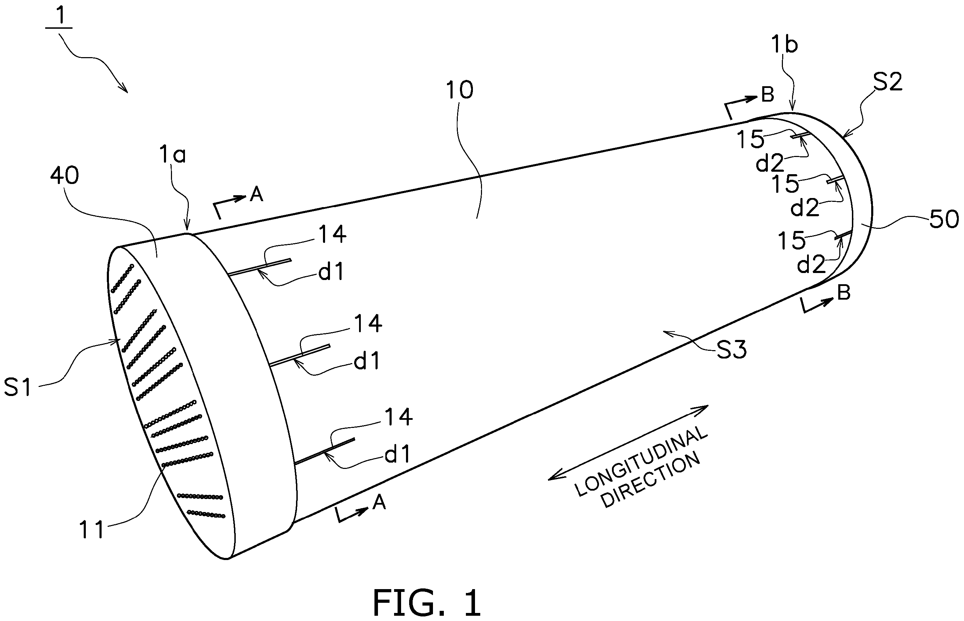

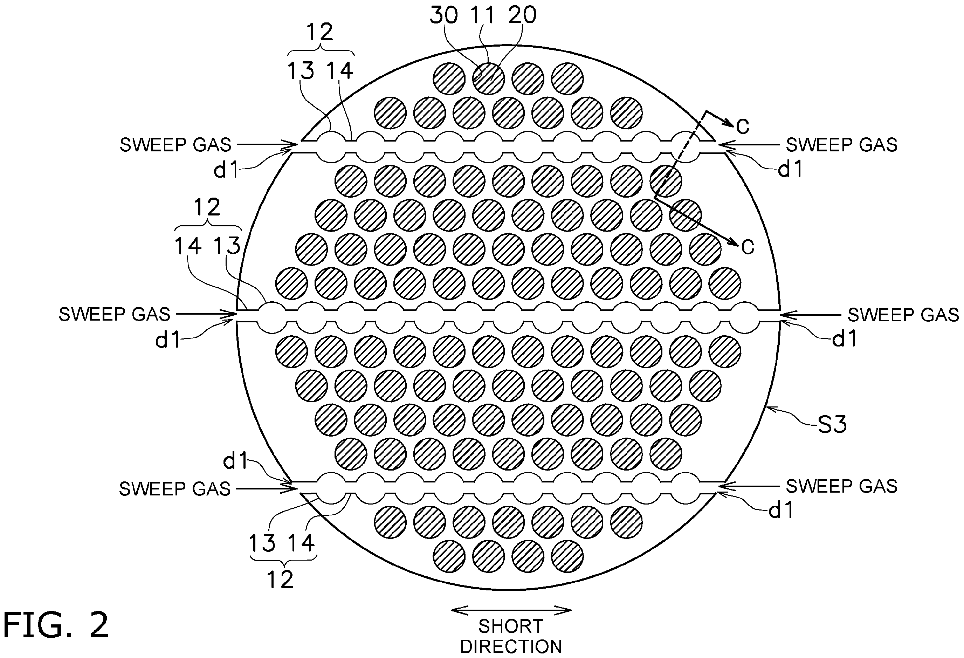

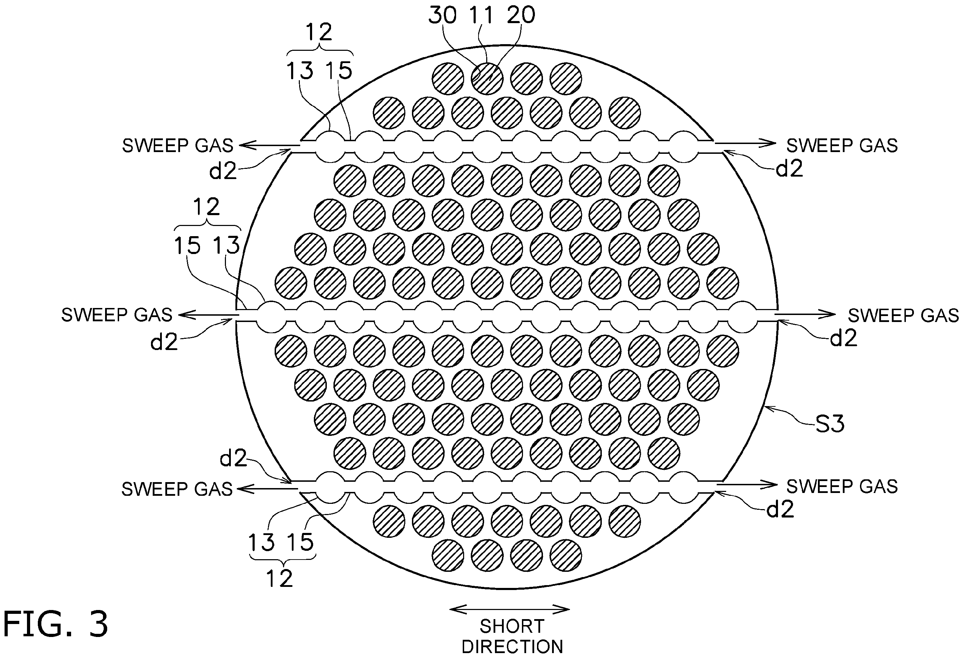

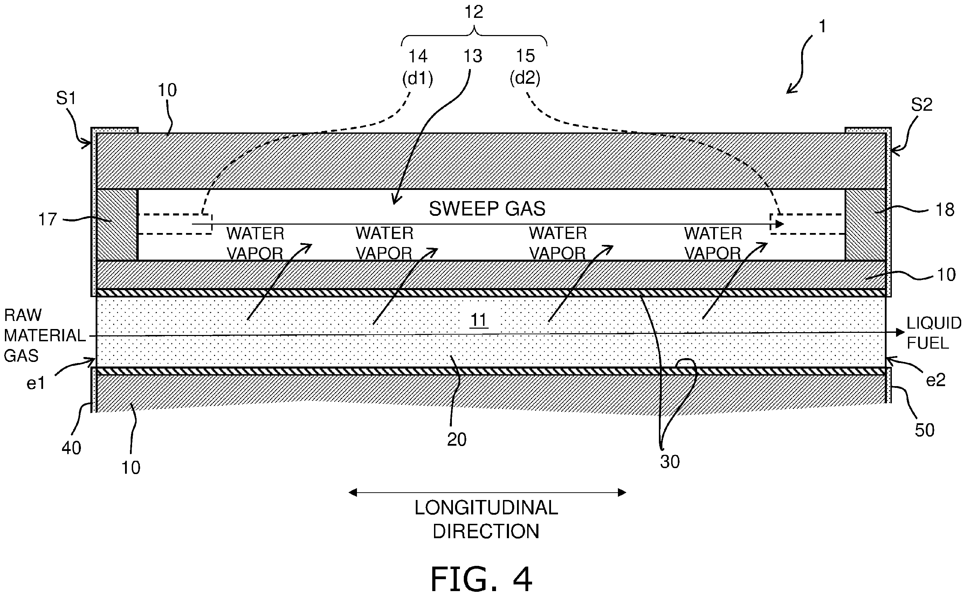

FIG. 1 is a perspective view of a reactor 1. FIG. 2 is a cross-sectional view taken along A-A in FIG. 1. FIG. 3 is a cross-sectional view taken along B-B in FIG. 1. FIG. 4 is a cross-sectional view taken along C-C in FIG. 2.

A reactor 1 is a so-called membrane reactor used to convert a raw material gas to a liquid fuel. The raw material gas contains at least hydrogen and carbon oxide. As the carbon oxide, at least one of carbon monoxide and carbon dioxide can be used. The raw material gas may be a so-called synthetic gas (syngas). The liquid fuel is a fuel that is in a liquid state at normal temperature and pressure, or a fuel that can be liquidized at normal temperature and pressurized state. Examples of the liquid fuel that is in a liquid state at normal temperature and pressure may include methanol, ethanol, liquid fuels represented by CₙH₂₍ₘ₋₂ₙ₎ (m is an integer less than 90, and n is an integer less than 30), and mixtures thereof. Examples of the fuel that can be liquidized at normal temperature and pressurized state include, for example, propane, butane, and mixtures thereof.

For example, reaction formula (1) for synthesizing methanol by catalytically hydrogenating a raw material gas containing carbon dioxide and hydrogen in the presence of a catalyst is as follows.

CO₂ + 3H₂ ↔ CH₃OH + H₂O (1)

The above reaction is an equilibrium reaction, and in order to increase both the conversion efficiency and the reaction rate, it is preferable to carry out the reaction at high temperature and high pressure (for example, 180°C or higher and 2 MPa or higher). The liquid fuel is in a gaseous state when it is synthesized, and is kept in a gaseous state at least until it flows out of the reactor 1. The reactor 1 preferably has heat resistance and pressure resistance suitable for desired synthesis conditions of the liquid fuel.

As shown in FIG. 1, the reactor 1 is formed in a monolith shape. A monolith means a shape including a plurality of holes that pass through in a longitudinal direction, and is a concept including a honeycomb. The reactor 1 extends in the longitudinal direction. The reactor 1 is formed in a columnar shape. Although the reactor 1 is formed in a circular columnar shape in the present embodiment, the outer shape of the reactor 1 is not particularly limited.

The reactor 1 includes a first end portion 1a and a second end portion 1b. The first end portion 1a is, when the reactor 1 is equally divided into five in the longitudinal direction, a portion extending to two-fifths from one end of the reactor 1. The second end portion 1b is, when the reactor 1 is equally divided into five in the longitudinal direction, a portion extending to two-fifths from the other end of the reactor 1. In the present embodiment, the first end portion 1a of the reactor 1 is on a side to which the raw material gas flows in, and the second end portion 1b of the reactor 1 is on a side from which the liquid fuel flows out.

The reactor 1 includes a first end face S1, a second end face S2, and a side face S3. The first end face S1 is an end face on the first end potion 1a side. The second end face S2 is an end face on the second end potion 1b side. The first end face S1 is provided on the opposite side to the second end face S2. The side face S3 is continuous with outer edges of the first end face S1 and the second end face S2.

As shown in FIGS. 1 to 4, the reactor 1 includes a porous support body 10, catalysts 20, separation membranes 30, a first seal portion 40, and a second seal portion 50.

The porous support body 10 is a columnar body extending in the longitudinal direction of the reactor 1. The porous support body 10 is constituted by a porous material.

As the porous material, a ceramic material, a metal material, a resin material, or the like can be used, and a ceramic material is particularly preferable. As an aggregate of the ceramic material, for example, at least one of alumina (Al₂O₃), titania (TiO₂), mullite (Al₂O₃-SiO₂), potsherd, cordierite (Mg₂Al₄Si₅O₁₈) can be used. As an inorganic binder for the ceramic material, for example, at least one of titania, mullite, readily sinterable alumina, silica, glass frit, a clay mineral, and readily sinterable cordierite can be used. However, the ceramic material does not need to contain an inorganic binder.

As shown in FIGS. 2 and 3, the porous support body 10 includes a number of first flow paths 11 and a plurality of second flow paths 12.

As shown in FIG. 4, the first flow paths 11 are formed in the longitudinal direction of the reactor 1. The first flow paths 11 are located on a non-permeation side of the separation membranes 30. The raw material gas is caused to flow through the first flow paths 11. The first flow paths 11 are through holes. The first flow paths 11 are open in the first end face S1 and the second end face S2 of the reactor 1. The first flow paths 11 each include an inflow port e1 of the raw material gas, formed in the first end face S1, and an outflow port e2 of the liquid fuel, formed in the second end face S2.

The catalysts 20 are respectively disposed in the first flow paths 11. The number, position, shape, and the like of the first flow paths 11 can be changed as appropriate.

The second flow paths 12 are located on a permeation side of the separation membranes 30. A sweep gas for sweeping the water vapor that has permeated through the separation membranes 30 is caused to flow through the second flow paths 12. An inert gas (e.g., nitrogen), air, or the like can be used as the sweep gas. In the present embodiment, which is a heat generation reaction, the temperature of the sweep gas is lower than an operation temperature of the reactor 1. The number, position, shape, and the like of the second flow paths 12 can be changed as appropriate.

Here, as shown in FIGS. 2 and 3, the second flow paths 12 are each formed by a plurality of cells 13, an inflow slit 14, and an outflow slit 15.

The plurality of cells 13 are arranged in a row along a short direction (a direction orthogonal to the longitudinal direction) of the reactor 1. As shown in FIG. 4, the cells 13 are formed along the longitudinal direction of the reactor 1. Both ends of each cell 13 are respectively sealed by first and second opening seal portions 17 and 18. The first and second opening seal portions 17 and 18 can be constituted by the above porous material.

As shown in FIG. 1, the inflow slits 14 are formed at the first end portion 1a in the longitudinal direction of the reactor 1. As shown in FIG. 2, the inflow slits 14 are formed along the short direction of the reactor 1. The inflow slits 14 pass through the plurality of cells 13. Both ends of the inflow slits 14 are open in the side face S3. The inflow slits 14 each include a pair of inflow ports d1 formed in the side face S3. The pair of inflow ports d1 correspond to one end of the second flow paths 12 in the longitudinal direction.

As shown in FIG. 1, outflow slits 15 are formed in the second end portion 1b of the reactor 1 in the longitudinal direction. As shown in FIG. 3, the outflow slits 15 are formed along the short direction of the reactor 1. The outflow slits 15 pass through the plurality of cells 13. Both ends of the outflow slits 15 are open in the side face S3. The outflow slits 15 each include a pair of outflow ports d2 formed in the side face S3. The pair of outflow ports d2 correspond to the other ends of the second flow paths 12 in the longitudinal direction.

The catalysts 20 are respectively disposed in the first flow paths 11. Although the catalysts 20 preferably fill the respective first flow paths 11, the catalysts 20 may be respectively disposed on the surfaces of the separation membranes 30 in a layered manner. As represented by the above formula (1), the catalysts 20 promote the conversion reaction of the raw material gas to the liquid fuel.

As the catalysts 20, a known catalyst suitable for the conversion reaction to a desired liquid fuel can be used. Examples of the catalysts 20 include metal catalysts (copper, palladium, and the like), oxide catalysts (zinc oxide, zirconia, gallium oxide, and the like), and composite catalysts thereof (copper-zinc oxide, copper-zinc oxide-alumina, copper-zinc oxide-chromium oxide-alumina, copper-cobalt-titania, catalysts obtained by modifying these with palladium, and the like).

The separation membranes 30 are supported by the porous support body 10. The separation membranes 30 surround the respective first flow paths 11. The separation membranes 30 are disposed between the first flow paths 11 and the second flow paths 12.

The separation membranes 30 are permeable to water vapor, which is one of the products of the conversion reaction of the raw material gas to a liquid fuel. In this manner, by utilizing the equilibrium shift effect, the reaction equilibrium of the above formula (1) can be shifted to the product side.

The separation membrane 30 preferably has a water vapor permeability coefficient of 100 nmol/(s·Pa·m²) or more. The water vapor permeability coefficient can be determined using a known method (see Ind. Eng. Chem. Res., 40, 163-175 (2001)).

The separation membrane 30 preferably has a separation factor of 100 or more. The greater the separation factor is, the more permeable the separation membrane 112 is to water vapor, and the less permeable it is to components other than water vapor (e.g., hydrogen, carbon dioxide, and liquid fuel). The separation factor can be determined using a known method (see FIG. 1 of "Separation and Purification Technology 239 (2020) 116533").

An inorganic membrane can be used as the separation membrane 30. The inorganic membrane is preferable because it has heat resistance, pressure resistance, and water vapor resistance. Examples of the inorganic membrane include a zeolite membrane, a silica membrane, an alumina membrane, and a composite membrane thereof. In particular, an LTA zeolite membrane having a molar ratio (Si/Al) of a silicon element (Si) and an aluminum element (Al) of 1.0 or more and 3.0 or less is suitable because of its excellent water vapor permeability.

As shown in FIG. 1, a first seal portion 40 covers the first end face S1 and part of the side face S3 of the porous support body 10. The first seal portion 40 suppresses entry of the raw material gas into the porous support body 10. As shown in FIG. 4, the first seal portion 40 is formed so as not to close the inflow ports e1 of the first flow paths 11. The first seal portion 40 covers a first opening seal portion 17. The first seal portion 40 can be constituted by glass, metal, rubber, resin, or the like.

As shown in FIG. 1, the second seal portion 50 covers part of the second end face S2 and the side face S3 of the porous support body 10. The second seal portion 50 suppresses entry of the liquid fuel into the porous support body 10. As shown in FIG. 4, the second seal portion 50 is formed so as not to close the outflow ports e2 of the first flow paths 11. The second seal portion 50 covers a second opening seal portion 18. The second seal portion 50 can be constituted by glass, metal, rubber, resin, or the like.

### Liquid Fuel Synthesis Method Using Reactor 1

A liquid fuel synthesis method using the reactor 1 will be described with reference to FIG. 4.

The liquid fuel synthesis method using the reactor 1 includes a step of causing the sweep gas to flow through the second flow paths 12 provided on the permeation side of the separation membranes 30, while causing the raw material gas to flow through the first flow paths 11 provided on the non-permeation side of the separation membranes 30.

The raw material gas flows into the first flow paths 11 through the inflow ports e1 of the first flow paths 11. In the first flow paths 11, water vapor is generated together with the liquid fuel in accordance with the above formula (1). The synthesized liquid fuel flows out through the outflow ports e2 of the first flow paths 11. Water vapor, which is one of the products, sequentially permeates through the separation membranes 30 and the porous support body 10, and then moves to the second flow paths 12. Note that the liquid fuel flowing out through the outflow ports e2 may be mixed with a residual raw material gas that was not used in the conversion reaction, water vapor, which is one of the products of the conversion reaction, and the like.

After flowing in through the inflow ports d1 of the inflow slits 14, the sweep gas flows into the cells 13 through the inflow slits 14. Next, the sweep gas that has flowed into the cells 13 through the inflow slits 14 takes in water vapor that has permeated through the separation membranes 30, and flows through the cells 13 toward the outflow slits 15 while absorbing the reaction heat generated by the conversion reaction. The sweep gas that has reached the outflow slits 15 flows out through the outflow ports d2 of the outflow slits 15.

As shown in FIG. 4, in the present embodiment, in the side view of the separation membranes 30, the direction in which the sweep gas flows through the second flow paths 12 is the same as the direction in which the raw material gas flows through the first flow paths 11. That is, the sweep gas flowing through the second flow paths 12 flows in the direction parallel with the direction in which the raw material gas flows through the first flow paths 11.

Note that in the side view of the separation membranes 30, the direction in which the sweep gas flows through the second flow paths 12 may be opposite to the direction in which the raw material gas flows through the first flow paths 11. That is, the sweep gas flowing through the second flow paths 12 may flow in the direction opposite to the direction in which the raw material gas flows through the first flow paths 11.

### Reactor Module 2

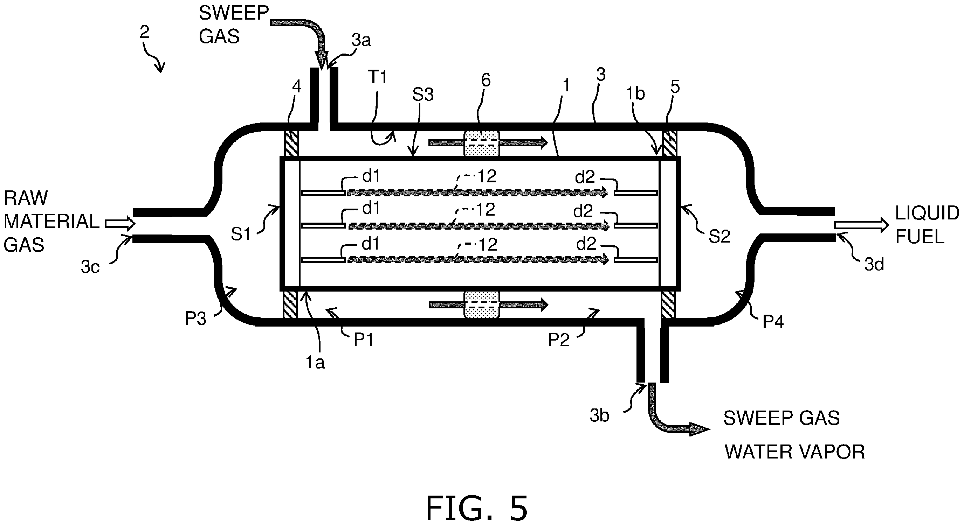

Next, a reactor module 2 according to the embodiment will be described. FIG. 5 is a transparent side view of the reactor module 2.

As shown in FIG. 5, the reactor module 2 is provided with the above monolith-type reactor 1, a housing 3, an annular first seal portion 4, an annular second seal portion 5 and an annular flow rate adjustment unit 6.

The housing 3 is constituted by a metal material such as a stainless steel. The housing 3 houses the reactor 1. The housing 3 includes a sweep gas supply port 3a, a sweep gas exhaust port 3b, a raw material gas supply port 3c and a liquid fuel exhaust port 3d. A space inside the housing 3 is partitioned into a first space P1 to a fourth space P4 by the first seal portion 4, the second seal portion 5, and the flow rate adjustment unit 6.

The first space P1 is a space between the first seal portion 4 and the flow rate adjustment unit 6. Sweep gas inflow ports d1 formed in the side face S3 of the reactor 1 are open to the first space P1. The sweep gas supply port 3a for supplying the sweep gas to the first space P1 is open to the first space P1. The second space P2 is a space between the second seal portion 5 and the flow rate adjustment unit 6. Sweep gas outflow ports d2 formed in the side face S3 of the reactor 1 are open to the second space P2. The sweep gas exhaust port 3b for discharging the sweep gas from the second space P2 is open to the second space P2. The first space P1 and the second space P2 are partitioned by the flow rate adjustment unit 6.

The raw material gas supply port 3c for supplying the raw material gas to the third space P3 is open to the third space P3. A raw material gas inflow port e1 (see FIG. 4) formed in the first end face S1 of the reactor 1 is open in the third space P3. The liquid fuel exhaust port 3d for discharging the liquid fuel from the fourth space P4 is open to the fourth space P4. A liquid fuel outflow port e2 (see FIG. 4) formed in the second end face S2 of the reactor 1 is open to the fourth space P4. The first space P1 and the third space P3 are partitioned by the first seal portion 4, and the second space P2 and the fourth space P4 are partitioned by the second seal portion 5.

The first seal portion 4 has an annular shape. The first seal portion 4 fixes the first end portion 1a of the reactor 1 to the housing 3. The first seal portion 4 is connected to the side face S3 of the reactor 1 and the inner face T1 of the housing 3. The first seal portion 4 seals a space between the first end portion 1a of the reactor 1 and the housing 3. Examples of the constituent materials of the first seal portion 4 include glass, silver solder, solder, and inorganic adhesive.

The second seal portion 5 has an annular shape. The second seal portion 5 fixes the second end portion 1b of the reactor 1 to the housing 3. The second seal portion 5 is connected to the side face S3 of the reactor 1 and an inner face T1 of the housing 3. Since the fourth space P4 side of the second seal portion 5 is exposed to a high temperature liquid fuel and water vapor, resistance against chemical load of the high temperature liquid fuel and resistance against water vapor are required in the constituent materials of the second seal portion 5. Examples of the constituent materials of the second seal portion 5 include glass, silver solder, solder, and inorganic adhesive. Rubber and plastic are not suitable for the constituent materials of the second seal portion 5.

The flow rate adjustment unit 6 has an annular shape. The flow rate adjustment unit 6 is disposed between the first seal portion 4 and the second seal portion 5 in the longitudinal direction. The flow rate adjustment unit 6 partitions between the first space P1 and the second space P2.

The flow rate adjustment unit 6 has air permeability. The flow rate adjustment unit 6 can cause the sweep gas to pass through from the first space P1 to the second space P2. The flow rate adjustment unit 6 is a member for adjusting the flow rate of the sweep gas flowing from the first space P1 to the second space P2 via the flow rate adjustment unit 6. The air permeability of the flow rate adjustment unit 6 can be adjusted by changing the porosity of the material constituting the flow rate adjustment unit 6, the width of the flow rate adjustment unit 6 in the longitudinal direction, or the number of flow rate adjustment units 6.

The flow rate adjustment unit 6 can be constituted by the porous material. The porous material can be constituted by exfoliated graphite, a porous rubber, a porous resin, or the like.

The sweep gas is supplied to the first space P1 through the sweep gas supply port 3a. Some of the sweep gas passes through the flow rate adjustment unit 6 from the first space P1 to the second space P2. The rest of sweep gas flows into the second flow path 12 through the inflow port d1 of the reactor 1. The sweep gas that has taken in water vapor and absorbed the reaction heat in the second flow path 12 flows out to the second space P2 through the outflow port d2 of the reactor 1. The sweep gas that has passed through the flow rate adjustment unit 6 and the sweep gas that has passed through the second flow path 12 join together in the second space P2 and are discharged to the outside through the sweep gas exhaust port 3b.

In this manner, the reactor 1 can be cooled from the outside using the sweep gas passing through the flow rate adjustment unit 6, as well as from the inside using the sweep gas flowing through the second flow paths 12. In particular, due to the sweep gas passing through the flow rate adjustment unit 6, the sweep gas can be inhibited from being retained near the flow rate adjustment unit 6, and thus the cooling efficiency from the outside can be improved without providing a special structure. Accordingly, since the reaction heat generated due to the conversion reaction can be efficiently removed, the conversion efficiency can be further improved.

Also, as shown in FIG. 5, in the side view of the reactor 1, the direction in which the sweep gas flows from the first space P1 to the second space P2 via the flow rate adjustment unit 6 is the same as the direction in which the sweep gas flows through the second flow paths 12. Accordingly, the reactor 1 can be cooled from the outside using the relatively cool sweep gas that has not passed through the inside of the reactor 1, and thus the cooling efficiency from the outside can be further improved.

Note that, in FIG. 5, the sweep gas supply port 3a and the sweep gas exhaust port 3b formed in the housing 3 are disposed on a straight line intersecting the axial center of the reactor 1 in the cross-sectional view. In this manner, since the lengths of the flow paths of the sweep gas that flows from the sweep gas supply port 3a to the sweep gas exhaust port 3b through the second flow paths 12 can be made the same, uneven flow of the sweep gas can be suppressed. Note that the positional relationship between the sweep gas supply port 3a and the sweep gas exhaust port 3b can be changed as appropriate.

### Modification of Embodiment

Although an embodiment of the present invention has been described above, the present invention is not limited to the above embodiment, and various modifications can be made without departing from the gist of the invention.

### Modification 1

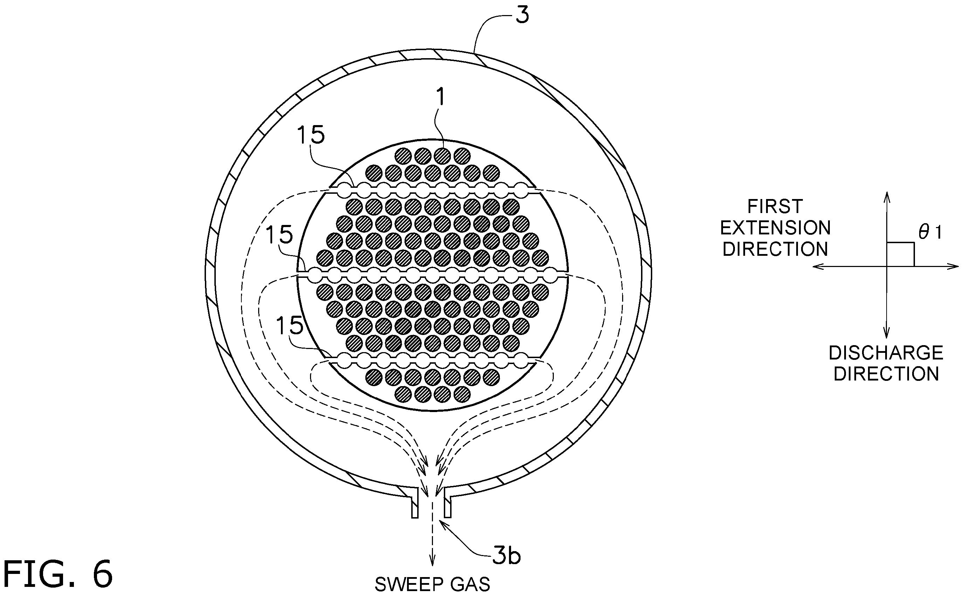

FIG. 6 is a cross-sectional view of a reactor module 2 shown in FIG. 5. FIG. 6 shows a cross-section perpendicular to the axial center of the reactor 1.

As shown in FIG.6, a first extending direction in which the outflow slits 15 extend in the reactor 1 is preferably inclined or orthogonal with respect to a sweep gas discharging direction in which the sweep gas is discharged to the outside through the sweep gas exhaust port 3b. Specifically, an angle θ1 of the first extending direction with respect to the discharging direction is preferably 45° or more and 135° or less. In this manner, uneven flow of the gas from the opening portions on both sides of the outflow slits 15 to the sweep gas exhaust port 3b can be suppressed, and thus uneven flow of the sweep gas can be suppressed.

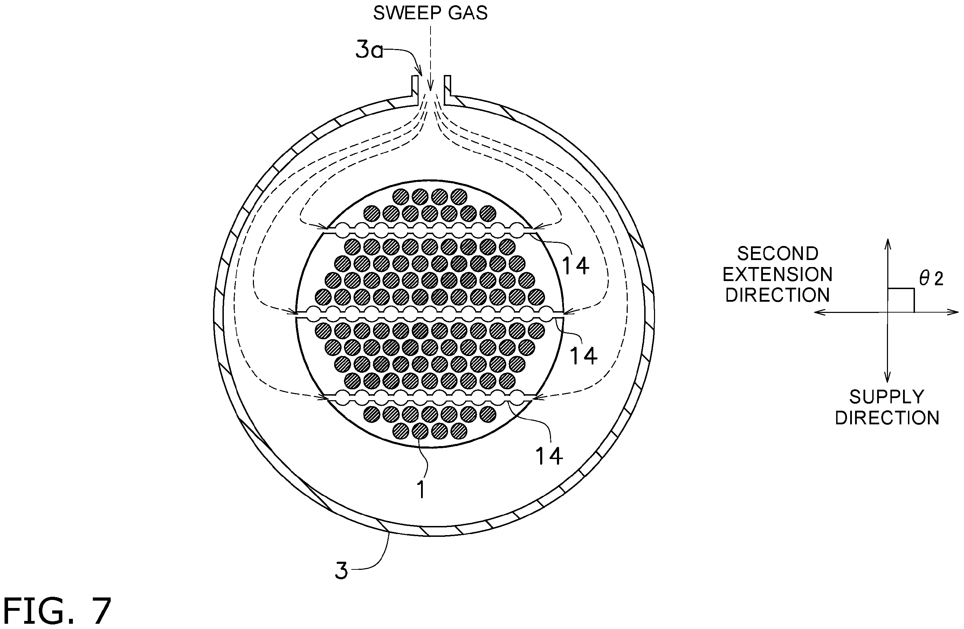

FIG. 7 is a cross-sectional view of the reactor module 2 shown in FIG. 5. FIG. 7 shows a cross section perpendicular to the axial center of the reactor 1.

As shown in FIG.7, a second extending direction in which the inflow slits 14 extend in the reactor 1 is preferably inclined or orthogonal with respect to a sweep gas supply direction in which the sweep gas is supplied through the sweep gas supply port 3a. Specifically, an angle θ2 of the second extending direction with respect to the supply direction is preferably 45° or more and 135° or less. In this manner, uneven flow of the gas from the sweep gas supply port 3a to opening portions on both sides of the inflow slits 14 can be suppressed, and thus uneven flow of the sweep gas can be suppressed.

### Modification 2

Although the separation membrane 30 is permeable to water vapor, which is a product of the conversion reaction of the raw material gas to the liquid fuel in the above embodiment, the present invention is not limited thereto. The separation membrane 30 may be permeable to the liquid fuel itself, generated through the conversion reaction of the raw material gas to the liquid fuel. In this case as well, the reaction equilibrium of the above formula (1) can be shifted to the product side.

Also, when the separation membrane 30 is permeable to the liquid fuel, even when generating the liquid fuel through a reaction in which no water vapor is generated (e.g., H₂ + CO ↔ CH₃OH), the reaction equilibrium can be shifted to the product side.

### Modification 3

Although the air-permeable annular flow rate adjustment unit 6 is constituted by the porous material in the above embodiment, there is no limitation thereto. The flow rate adjustment unit 6 need only have a structure capable of causing the sweep gas to pass through from the first space P1 to the second space P2.

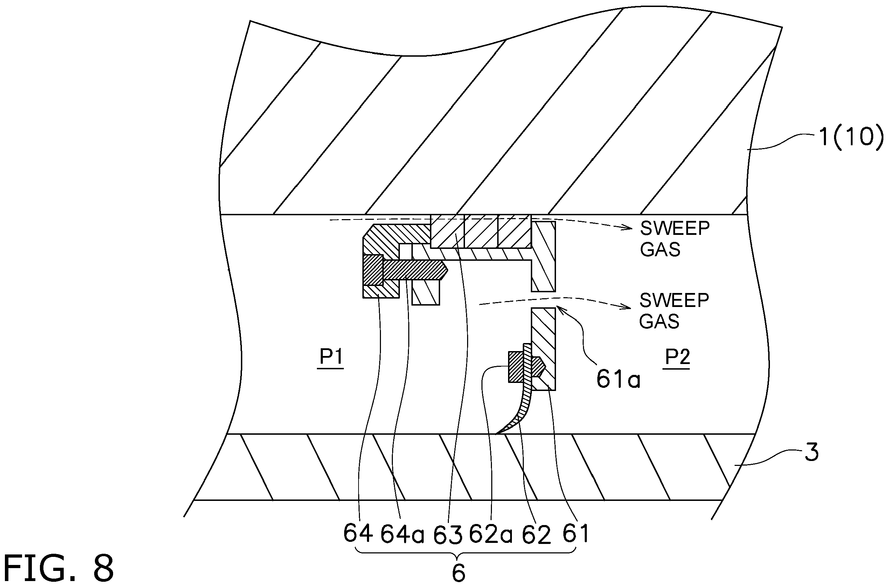

Here, FIG. 8 is a cross-sectional view of the flow rate adjustment unit 6. As shown in FIG. 8, the flow rate adjustment unit 6 can be constituted by a support member 61, a packing 62, a seal member 63, and a pressing member 64.

The support member 61 has an annular shape. The support member 61 is disposed surrounding the reactor 1 (porous support body 10). The support member 61 supports the packing 62 and the seal member 63. A vent hole 61a is formed in the support member 61. The vent hole 61a is in communication with the first space P1 and the second space P2. The sweep gas flows from the first space P1 to the second space P2 via the vent hole 61a. The flow rate of the sweep gas can be adjusted as appropriate by changing the size and shape of the vent hole 61a.

The packing 62 is an annular elastic member. The packing 62 is fixed to the support member 61 with a fastening member 62a. The packing 62 is disposed between the support member 61 and the housing 3. The packing 62 seals a gap between the support member 61 and the housing 3.

The seal member 63 is an annular gland packing. The seal member 63 can be constituted by, for example, exfoliated graphite. The seal member 63 is disposed between the support member 61 and the reactor 1 (porous support body 10). The seal member 63 is compressed by the pressing member 64. The air permeability of the seal member 63 can be adjusted by changing the pressing force of the pressing member 64. The sweep gas flows from the first space P1 to the second space P2 via the seal member 63. The flow rate of the sweep gas can be adjusted as appropriate by changing the compression force of the pressing member 64.

The pressing member 64 is fixed to the support member 61 by a fastening member 64a. The pressing member 64 presses the seal member 63. The pressing force of the pressing member 64 can be adjusted as appropriate by changing the fastening amount of the fastening member 64a.

In this manner, the flow rate adjustment unit 6 shown in FIG. 8 exhibits air permeability due to the vent hole 61a and the seal member 63 of the support member 61 functioning as the flow paths of the sweep gas.

Note that the flow rate adjustment unit 6 shown in FIG. 8 may have only one of the vent hole 61a and the seal member 63.

### Modification 4

Although a reactor module provided with a reactor has been described in the above embodiment, the present invention can also be applied to a separation membrane module provided with a separation filter. The separation filter has the same configuration as the reactor 1 according to the above embodiment, except that the separation filter has a separation membrane for separating a predetermined component from a mixed fluid.

In such a separation membrane module, in order to control the separation membrane at an appropriate temperature, the separation membrane is to be heated or cooled by causing the sweep gas to flow through the permeation side flow path in some cases. In this case, as in the above embodiment, in the side view of the separation filter, the direction in which the sweep gas flows from the first space P1 to the second space P2 via the flow rate adjustment unit 6 is set the same as the direction in which the sweep gas flows through the second flow paths 12. In this manner, the separation filter can be efficiently cooled or heated from the outside using the sweep gas that has not passed through the inside of the separation filter.

In the above embodiment, the temperature of the sweep gas is lower than the operation temperature of the reactor 1. However, in the separation membrane module, in order to cool the separation membrane, it is necessary to set the temperature of the sweep gas lower than the temperature of the separation filter. On the other hand, in order to heat the separation membrane, it is necessary to set the temperature of the sweep gas higher than the temperature of the separation filter.

### REFERENCE SIGNS LIST

- 1: Reactor
- 2: Reactor module
- 3: Housing
- 3a: Sweep gas supply port
- 3b: Sweep gas exhaust port
- 3c: Raw material supply port
- 3d: Liquid fuel exhaust port
- 4: First seal portion
- 5: Second seal portion
- 6: Flow rate adjustment unit
- 10: Porous support body
- 11: First flow path
- e1: Inflow port
- e2: Outflow port
- 12: Second flow path
- 13: Cell
- 14: Inflow slit
- d1: Inflow port
- 15: Outflow slit
- d2: Outflow port
- 20: Catalyst
- 30: Separation membrane
- 40: First seal portion
- 50: Second seal portion

## Claims

1. A reactor module comprising:
a monolith-type reactor extending in a longitudinal direction;
a housing configured to house the reactor;
an annular first seal portion configured to seal a space between the housing and a first end portion of the reactor;
an annular second seal portion configured to seal a space between the housing and a second end portion of the reactor; and
an air-permeable annular flow rate adjustment unit disposed between the first seal portion and the second seal portion in the longitudinal direction, wherein
the reactor includes a separation membrane permeable to a product of a conversion reaction of a raw material gas containing hydrogen and carbon oxide to a liquid fuel, a first flow path on a non-permeation side of the separation membrane, and a second flow path on a permeation side of the separation membrane,
the second flow path includes an inflow port open to a first space between the first seal portion and the flow rate adjustment unit, and an outflow port open to a second space between the second seal portion and the flow rate adjustment unit,
the housing includes a supply port for supplying a sweep gas to the first space, and an exhaust port for discharging the sweep gas from the second space, and
in a side view of the reactor, a direction in which the sweep gas flows from the first space to the second space via the flow rate adjustment unit is the same as a direction in which the sweep gas flows through the second flow path.

2. A liquid fuel synthesis method using the reactor module according to claim 1, the method comprising:
a step of supplying the sweep gas to the first space through the supply port, wherein
in a side view of the reactor, the direction in which the sweep gas flows from the first space to the second space via the flow rate adjustment unit is the same as the direction in which the sweep gas flows through the second flow path.

3. A separation membrane module comprising:
a monolith-type separation filter extending in a longitudinal direction;
a housing configured to house the separation filter;
an annular first seal portion configured to seal a space between the housing and a first end portion of the separation filter;
an annular second seal portion configured to seal a space between the housing and a second end portion of the separation filter; and
an air-permeable annular flow rate adjustment unit disposed between the first seal portion and the second seal portion in the longitudinal direction, wherein
the separation filter includes a separation membrane for separating a predetermined component from a mixed fluid, a first flow path on a non-permeation side of the separation membrane, and a second flow path on a permeation side of the separation membrane, and
the second flow path includes an inflow port open to a first space between the first seal portion and the flow rate adjustment unit, and an outflow port open to a second space between the second seal portion and the flow rate adjustment unit,
the housing includes a supply port for supplying a sweep gas to the first space, and an exhaust port for discharging the sweep gas from the second space, and
in a side view of the separation filter, a direction in which the sweep gas flows from the first space to the second space via the flow rate adjustment unit is the same as a direction in which the sweep gas flows through the second flow path.

4. A separation method for separating a predetermined component from a mixed fluid using the separation membrane module according to claim 3, the method comprising:
a step of supplying the sweep gas to the first space through the supply port, wherein
in a side view of the separation filter, a direction in which the sweep gas flows from the first space to the second space via the flow rate adjustment unit is the same as the direction in which the sweep gas flows through the second flow path.
